# EUROPEAN PATENT SPECIFICATION

(11) **EP 2 034 963 B1**
(45) Date of publication and mention of the grant of the patent: **10.08.2022**
(21) Application number: 07794902.2
(22) Date of filing: 16.05.2007
(51) Int. Cl.: A61K 9/127, A61K 9/00, A61K 9/06, A61K 47/34, A61K 41/00, A61P 43/00

(54) **GEL-FORMULATIONS OF HYDROPHOBIC PHOTOSENSITIZERS FOR MUCOSAL APPLICATIONS**
GELFORMULIERUNGEN HYDROPHOBER PHOTOSENSIBILISATOREN ZUM AUFTRAGEN AUF DIE SCHLEIMHAUT
FORMULATIONS DE GELS DE PHOTOSENSIBILISATEURS HYDROPHOBES POUR APPLICATIONS MUQUEUSES

(30) Priority: 18.05.2006 US 801493 P; 07.05.2007 US 800599
(43) Date of publication of application: 18.03.2009
(73) Proprietor: biolitec Unternehmensbeteiligungs II AG, 1030 Vienna (AT)
(72) Inventor: ALBRECHT, Volker, 14558 Nuthetal (DE); SCHEGLMANN, Dietrich, 07751 Jena (DE)
(74) Representative: Herrmann, Franz
(86) International application number: PCT/US2007/011676
(87) International publication number: WO 2007/136633

(56) References cited:
- US-A- 5 616 602
- US-A- 6 074 666
- US-A1- 2002 155 089
- US-A1- 2004 009 212
- US-A1- 2005 048 109
- US-B1- 6 416 740

## Description

### Background of the Invention

This application claims the benefit of U.S. Provisional Application Serial No. 60/801,493 filed May 18, 2006, and U.S. Full Application filled on May 7, 2007.

### 1. Field of the invention

The invention relates to a pharmaceutical composition useful in photodynamic therapy (PDT).

### 2. Information Disclosure Statement

For an effective drug treatment it is important to maintain the drug concentration for a certain period of time at the absorption site and thereby facilitate the uptake of the drug. Drug applied to the mucosal layer (i.e. covering the mucosal epithelial surface) undergoes fast elimination due to the effective clearance mechanism seen in these parts of the body, thus reducing the duration of the therapeutic effect and hence requiring frequent application. When frequent dosing is required, it can result in increased costs and may lead to decreased patient compliance. In an effort to overcome these shortcomings, researchers have looked at improving the delivery systems of pharmaceutical substances. Thus a suitable drug carrier with mucoadhesive properties can improve the effectiveness of drug transfer at the absorption site.

Mucoadhesive drug delivery systems are using the attraction between mucosa cells and the polymeric drug carrier. They provide localization of the drug at the specific body site and increase penetration efficiency of the drug. These features greatly enhance the bioavailability of drugs. Obviously, understanding of mucosa/polymer interactions in the physiological environment is essential for designing mucoadhesive delivery systems.

Drug carriers are frequently used to transport and deliver drugs through the body; they protect the drug against degradation and may extend the circulation time in the bloodstream. Normal cells can be protected from the toxic side effects of the drug, and the drug can be targeted to the site of action. Examples of such carrier are microparticles, drug-polymer conjugates, polymer micelles, liposomes and nanospheres.

Drug carriers can be used to delivery hydrophobic PS and their precursors. The medicament carrier can be composed of natural and or synthetic phospholipids which are capable of forming micelles/liposomes. Hydrophobic Photosensitizers and their precursors are insoluble in water, and therefore when administered they are absorbed by the body before being dissolved thus causing adverse side effects. Current attempts to delivery water-insoluble substance in medicaments mostly involve some form of encapsulation or solvent carriers other than water.

While formulating a hydrophobic composition for mucosal application, it is important to use substances which are able to solubilize hydrophobic substances stable at physiological conditions, and most of all are non-irritant to the mucosal cell layer.

Several bioadhesive drugs delivery systems based on a variety of polymers have been described in patents and in the literature. For example, U.S. Patent 6,387,408 by Illum et al. describes the use of adhesive material, 'adhesin' derived from bacteria or synthetic analog of the same to combine with a drug entrapped in liposome or emulsion to provide attachment to the gastrointestinal tract. Another example in U.S. Patent 6,428,813 by Akiyama et al. discloses an *anti-Helicobacter pylori* pharmaceutical agent with enhanced gastrointestinal mucosa-adherent composition to treat gastric and duodenal ulcers.

US Patent 6,582,720 by Inagi et al. also discusses a medicinal composition having adhering capacity to the mucosal layer of stomach and duodenum. Here the drug carrier attaches to the mucosal layer at acidic pH values. In Poly (acrylic acid) (PAA) the adhesive interaction is reduced at pH higher than 5, while mucoadhesive strength of poly (hydroxyethyl methacrylate) (PHEMA) is reduced at pH 1. U.S. Patent Application 2004/0009212 by Tsai describes a thermoresponsive mucoadhesive-carrier composition for topical delivery of PS useful in photodynamic therapy (PDT). US Patent Application 2002/0156062 by Boch et al, discloses use of water soluble microaggregates (e.g. micelles, liposome) for delivering hydrophobic (water insoluble) polypyrrolic macrocyle-based photosensitizers, however the formulation is not specific to mucosal administration.

US 2004/0009212 Al discloses a medicament-carrier composition which mainly comprises a mucoadhesive polymer and a thermoresponsive polymer. The medicament-carrier composition is especially suitable for use in topical delivery of 5-aminolevulinic acid (= ALA) for photodynamic diagnosis or therapy.

US 2005/0048109 Al discloses a pharmaceutical liposomal formulation for photodynamic therapy comprising a non-polar porphyrin photosensitizer and one or more phospholipids. The liposomal formulation provides therapeutically effective amounts of the photosensitizer for intravenous administration. The phospholipids may be modified by pegylation.

In the field of PDT, there is a continuing need for a drug delivery system that is simple, non-toxic, chemically inert, and economical and that can easily be used for formulating different types of photosensitizers. Formulations of hydrophobic photosensitizers are required not only to maintain the drug in relatively non-aggregate form, but also to achieve effective delivery to a target site. The formulation should be stable prior to administration, while displaying effective delivery and performance at the target site.

### Objectives and Brief Summary of the Invention

It is an object of this invention to provide an improved pharmaceutical formulation for hydrophobic photosensitizer which is mTHPC for topical application.

It is also the object of this invention to provide an innovative formulation of said hydrophobic PS which is suitable for mucosal application.

It is another objective of the present invention to incorporate said hydrophobic PS and their precursors into a liposomal vesicle of suitable size thus facilitating easy transport across the mucin layer.

It is a further objective of the invention to use one or more block copolymers or polymers with mucoadhesive property to improve interaction between mucosal layer and the medicament formulation.

Briefly stated, the present invention provides improved formulations of a hydrophobic photosensitizer which is mTHPC and its precursors which are suitable for mucosal administration. The formulation of the invention comprises said hydrophobic photosensitizer which has been incorporated into suitably sized liposomes. Additionally, these formulations include the incorporation of PS-enclosed liposomes into a block copolymer or polymer matrix.

The liposome of the present invention allows the hydrophobic photosensitizer to be incorporated into a thermogel or polymer matrix and thus promotes intimate contact between the formulation and the mucosal layer for enhanced drug absorption.

The above and other objects, features and advantages of the present invention will become apparent from the following description read in conjunction with the accompanying drawings.

### Brief Description of Figures

Figure 1 is a gel filtration curve of liposomal formulated mTHPC. Both, lipid components and mTHPC show the same distribution over all fractions collected.
Figure 2 depicts the penetration behavior of the gel formulation in cellulose matrix at 37°C, 100 %RH.

### Detailed Description of Preferred Embodiments

In this invention one or more block copolymer-liposome or polymer-liposome formulations are used, to aid the delivery of highly hydrophobic drug which is mTHPC. The block copolymer includes symmetrical triblock copolymers composed of polyethylene oxide (PEO) and polypropylene oxide (PPO). The polymer is selected from the group consisting of poly (acrylic acid) (PAA), carbopol, hydroxypropylmethylcellulose, poly(methyl-acrylate), hyaluronic acid and chitosan.

An ideal formulation should be nontoxic and non-irritant to the mucosal epithelium, should be inexpensive, and should have a broad specificity for solubilizing the water insoluble, and slightly soluble pharmaceutical agents, thereby offering flexibility of application with respect to improving the bioavailability of poorly water-soluble drugs and manipulating release profiles. Thus overcoming the problems in present drug delivery systems, the described formulations of a copolymer-liposome-drug-carrier shows good mucosal adherent property and high bioavailibility of the drug.

Given that the human body consists mostly of water, hydrophobic drugs are problematic when a patient's body tries to absorb them. For improving efficiency of delivery of hydrophobic PS and their precursor, one can envelope such compounds in a lipid structure termed liposome. The majority of photosensitizers of interest for PDT are hydrophobic. The tendency of highly hydrophobic photosensitizing drugs to undergo aggregation in contact with aqueous systems has a diminishing effect on the activation of the photosensitizer by light. Therefore, such drugs need to be administered in a formulation suitable to minimize aggregation in vivo.

The mucosal administration of pharmaceutical agents causes a therapeutic response only if significant dosages of the drug are enabled to permeate the absorption membrane-mucus layer. The drug permeation can be improved by using mucoadhesive polymers and permeation enhancers. The generation of novel drug formulation based on the knowledge of the absorption membrane can improve the bioavailability of mucosally applied drugs. The pharmaceutical agents mentioned above are hydrophobic photosensitizer and their precursors which are not soluble in water or are only slightly soluble in water. Delivering hydrophobic drugs to mucosal layer which is composed of 90% water as a main component requires a well designed drug formulation.

Drug delivery to the mucosal layer faces many problems as the mucosal layers are protected by effective clearance mechanisms. A drug delivery system comprising a mucoadhesive polymer can increase the contact time of the formulation at the mucosal surface thus enhancing the amount of the drug being absorbed.

The pathway by which the PS is taken up into cells depends on its chemical properties, size, charges and hydrophilic and hydrophobic properties. Furthermore, the type of the medicament carrier used for delivering the PS to the cell may also influence the intracellular accumulation and distribution. Therefore it is important to understand how these pharmaceutical compounds are internalized into the target cells.

Mucoadhesive delivery systems are able to adhere on the mucous layer covering the mucosal epithelium. These mucoadhesive properties are in many cases advantageous in order to enhance the permeation of PS through the mucosal absorption membrane by prolonging the residence time of the drug on the mucosa, which allows a sustained drug release at this site; thereby, prolonged period of drug uptake, and, subsequently a greater amount of total drug absorbed can be achieved. An intimate contact of the drug carrier to the absorption membrane can be guaranteed providing the basis for a high concentration gradient as the driving force for the drug uptake.

In one of the embodiment of the present invention an innovative formulation for hydrophobic PS which is mTHPC and its precursor are proposed, with improved targeting ability and enhanced photodynamic activity. The hydrophobic PS and PS precursors are packaged into a suitable sized (150 nm) liposomal vesicle. The size of the liposome is important for the diffusion process across the mucous layer, hydrophobic PS -loaded liposome of large size show poor diffusion across the mucin membrane, while reducing the size improves the diffusion across the cell layer. The hydrophobic PS which is lipid soluble is incorporated into a liposome bilayer. The presence of surface ligands such as monosialoganglioside or polyoxyethylene on the liposomal surface enhances liposome stability in the physiological environment.

The mucoadhesion of polymers to the mucosa is essential for their interaction with the membrane in order to achieve a permeation enhancing effect. To serve as mucoadhesive polymer, the polymers should possess some general physiochemical features such as suitable surface property for wetting mucus/mucosal tissue surfaces and sufficient flexibility to penetrate the mucous membrane. Most efficient mucoadhesive polymers have physiochemical properties that are mostly similar to mucus substrate.

Different classes of synthetic and natural polymers have been investigated for potential use as mucoadhesives. Examples of synthetic polymers are poly (acrylic acid) (PAA), carbopol, hydroxypropyl-methylcellulose, poly (methyl-acrylate) and natural polymers like hyaluronic acid and chitosan. In some cases copolymerization is required to improve the mucoadhesive property of the polymer. The use of block copolymer at the gel surface provides more detailed control on the gel-mucosa interaction.

In this invention copolymer-liposome based formulations that offer rapid dispersion and enhanced drug absorption are proposed. These copolymer liposome formulations appear to have the ability to deliver larger amounts of pharmaceutical composition into the mucosa than do traditional lotions and creams because they provide a better reservoir for a poorly soluble drug through their capacity for enhanced solubilization. The hydrophobic drugs are successfully formulated in a block copolymer known as pluronics. Pluronics are a family of symmetrical triblock copolymers composed of polyethylene oxide (PEO) and polypropylene oxide (PPO). Pluronics are used in pharmaceutical industry for emulsification, solubilization, dispersion, and as thickening, coating and wetting agents.

The present invention is further illustrated by the following examples, but is not limited thereby.

### Example 1:

Gel formulation (0.5mg mTHPC/ml; 19% (w/w) Lutrol and water) shows high solubility of the hydrophobic drug. (m-tetrahydroxyphenylchlorin (mTHPC) also referred as Temoporfin is a hydrophobic photosensitizer):

| | **drug content (A₆₅₀ₙₘ)** | |
|---|---|---|
| | **w/o filtration** | **0,2 µm filtration** |
| **Gel formulation (concentration 1)** | **0.48 ± 0.01** | **0.49 ± 0.05** |
| **Gel formulation (concentration 2)** | **0.536 ± 0.013** | **0.539 ± 0.014** |

The above table value shows that no aggregation of the drug has been observed.

### Example 2

### Localization of mTHPC within the liposomal bilayer of the formulation

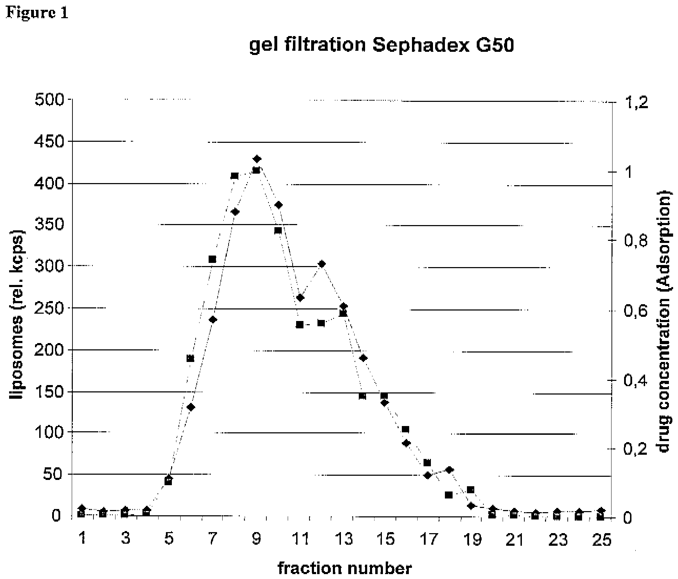

Gel filtration of liposomal formulation performed on Sephadex G50 columns. As shown in Figure 1, lipids and mTHPC show the same distribution over all fractions indicating a physically interaction of both components i.e. integration of mTHPC into the lipid bilayer.

### Example 3

### Stability of the gel formulation

Particle size of liposomes is stable during thermo setting of the gel matrix as given in the table below.

| | **Particle size (nm)** | **polydispersity index** |
|---|---|---|
| **drug loaded gel formulation, before temperature shift to 37°C** | **142.7 ± 0.2** | **0.123 ± 0.005** |
| **drug loaded gel formulation, after temperature shift to 37°C** | **141.4 ± 0.6** | **0.137 ± 0.005** |

### Example 4

Stability of the ready to use gel formulation at room temperature(drug concentration 0.5 mg/ml).

| **particle size (nm)** | **drug (mg/ml)** | **storage time (days)** | **PI** |
|---|---|---|---|
| **124** | **0.5** | **1** | **0.64** |
| **122** | **0.5** | **6** | **0.63** |
| **102** | **0.5** | **19** | **0.80** |
| **110** | **0.5** | **36** | **0.62** |
| **117** | **0.5** | **104** | **0.80** |
| **112** | **0.5** | **180** | **0.60** |
| **117** | **0.5** | **280** | **0.61** |
| **108** | **0.5** | **418** | **0.69** |
| **118** | **0.5** | **467** | **0.58** |

The particle size of the loaded formulation increased only slightly over the period of storage as observed from the table, indicating a high physical stability at the storage temperature and condition.

### Example 5:

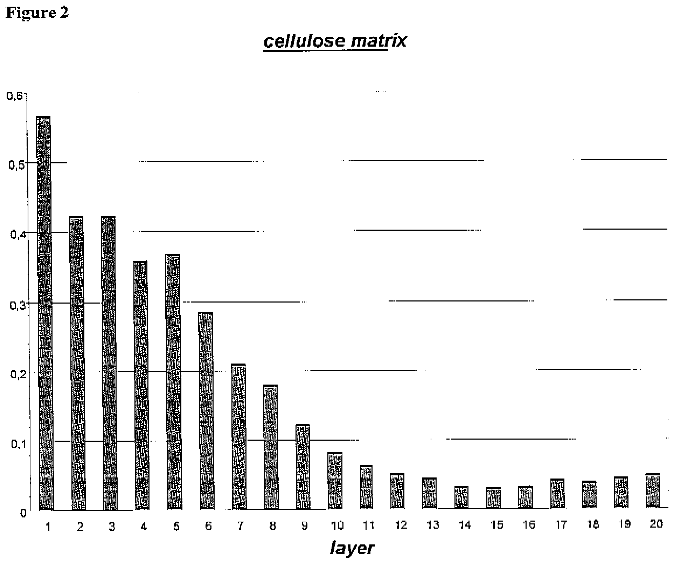

Figure 2 illustrates the penetration behavior of the gel formulation of mTHPC (0.5 mg drug/ml) which was administrated to cellulose matrix at 37°C, 100% RH. The drug concentrations are measured after 16 hours of incubation. Each layer represents 0.25 mm of depth and layer 1 is the site of application. From figure 2 it is seen that the drug penetration decreases as the number layers increases.

## Claims

1. A chemical composition formulation for the delivery of a hydrophobic drug across a mucus coated membrane, comprising:
a carrier for solubilizing said drug, said carrier being chemically stable in an environment of said mucus coated membrane, said carrier being a non-mucosal irritant, and said carrier being a mucoadhesive; and
a lipid soluble hydrophobic photosensitizer or a precursor of such photosensitizer being lipid soluble;
wherein the carrier comprises a liposomal vesicle and a block copolymer for mucoadhesion,
wherein said block copolymer includes symmetrical triblock copolymers composed of polyethylene oxide (PEO) and polypropylene oxide (PPO), or a polymer for mucoadhesion selected from the group consisting of poly (acrylic acid) (PAA), carbopol, hydroxypropyl-methylcellulose, poly (methyl-acrylate), hyaluronic acid and chitosan;
wherein the photosensitizer or precursor of the photosensitizer is packaged into said liposomal vesicle; and
wherein the photosensitizer is temoporfin (mTHPC).

2. The chemical composition formulation according to claim 1,
wherein said carrier comprises a phospholipid.

3. The chemical composition formulation according to claim 1,
further including a permeation enhancer.

4. The chemical composition formulation according to any one of claims 1 to 3,
further including ligands on the liposomal surface.

5. The chemical composition formulation according to claim 4,
wherein said ligands are monosialoganglioside or polyoxyethylene.

6. The chemical composition formulation according to any one of claim 1 to 5,
wherein said liposome has an initial diameter of less than or about 150 nm.

7. The chemical composition formulation according to any one of the preceding claims for use in drug delivery through a mucosal membrane using a thermogel or polymer matrix formulation
comprising the steps of:
a) providing a chemical composition formulation as defined any one of the preceding claims; and
b) applying said chemical composition formulation to a mucosal membrane.

8. The chemical composition formulation for use according to claim 7,
wherein said formulation is used to treat cell dysplasia, cancer or other disease conditions.

## Patentansprüche

1. Formulierung einer chemischen Zusammensetzung für die Abgabe eines hydrophoben Arzneimittels durch eine schleimbeschichtete Membran mit:
einem Träger zur Lösung des Arzneimittels, wobei der Träger in der Umgebung der schleimbeschichteten Membran chemisch stabil ist, wobei der Träger ein nicht schleimhautreizendes Mittel ist und wobei der Träger ein Mukoadhäsiv ist; und
einem lipidlöslichen hydrophoben Photosensibilisator oder einem Vorläufer eines solchen Photosensibilisators, der lipidlöslich ist;
wobei der Träger ein liposomales Vesikel und ein Blockcopolymer für die Mukoadhäsion aufweist, wobei das Blockcopolymer symmetrische Triblockcopolymere, die aus Polyethylenoxid (PEO) und Polypropylenoxid (PPO) zusammengesetzt sind, oder ein Polymer für die Mukoadhäsion umfasst, das aus der Gruppe ausgewählt ist, die aus Poly(acrylsäure) (PAA), Carbopol, Hydroxypropylmethylcellulose und Poly(methylacrylat), Hyaluronsäure und Chitosan besteht;
wobei der Photosensibilisator oder der Vorläufer des Photosensibilisators in das liposomale Vesikel verpackt ist; und
wobei der Photosensibilisator Temoporfin (mTHPC) ist.

2. Formulierung einer chemischen Zusammensetzung nach Anspruch 1,
wobei der Träger ein Phospholipid umfasst.

3. Formulierung einer chemischen Zusammensetzung nach Anspruch 1,
die ferner einen Permeationsverstärker enthält.

4. Formulierung einer chemischen Zusammensetzung nach einem der Ansprüche 1 bis 3,
die ferner Liganden auf der liposomalen Oberfläche enthält.

5. Formulierung einer chemischen Zusammensetzung nach Anspruch 4,
wobei die Liganden Monosialogangliosid oder Polyoxyethylen sind.

6. Formulierung einer chemischen Zusammensetzung nach einem der Ansprüche 1 bis 5,
wobei das Liposom einen Anfangsdurchmesser von weniger als oder etwa 150 nm aufweist.

7. Formulierung einer chemischen Zusammensetzung nach einem der vorhergehenden Ansprüche zur Verwendung bei der Arzneimittelabgabe durch eine Schleimhautmembran unter Verwendung einer Thermogel-Matrixformulierung mit den folgenden Schritten:
a) Bereitstellen einer Formulierung einer chemischen Zusammensetzung, wie in einem der vorhergehenden Ansprüche definiert; und
b) Aufbringen der Formulierung der chemischen Zusammensetzung auf eine Schleimhautmembran.

8. Formulierung einer chemischen Zusammensetzung zur Verwendung gemäß Anspruch 7,
wobei die Formulierung zur Behandlung von Zelldysplasie, Krebs oder anderen Krankheitszuständen verwendet wird.

## Revendications

1. Formulation de composition chimique destinée à l'administration d'un médicament hydrophobe en travers d'une membrane enduite de mucus, comprenant :
un vecteur pour solubiliser ledit médicament, ledit vecteur étant stable chimiquement dans un environnement de ladite membrane enduite de mucus, ledit vecteur étant un irritant non muqueux, et ledit vecteur étant un muco-adhésif ; et
un photosensibilisateur hydrophobe soluble dans un lipide ou un précurseur d'un tel photosensibilisateur qui est soluble dans un lipide ;
dans laquelle le vecteur comprend une vésicule liposomale et un copolymère séquencé pour la muco-adhésion,
dans laquelle ledit copolymère séquencé inclut des copolymères triblocs symétriques composés d'oxyde de polyéthylène (PEO) et d'oxyde de polypropylène (PPO), ou un polymère pour la muco-adhésion sélectionné parmi le groupe constitué de l'acide poly(acrylique) (PAA), du carbopol, de l'hydroxypropylméthylcellulose et du poly(méthylacrylate), de l'acide hyaluronique et du chitosan ;
dans laquelle le photosensibilisateur ou précurseur du photosensibilisateur est emballé dans ladite vésicule liposomale ; et
dans laquelle le photosensibilisateur est la témoporfine (mTHPC).

2. Formulation de composition chimique selon la revendication 1,
dans laquelle ledit vecteur comprend un phospholipide.

3. Formulation de composition chimique selon la revendication 1,
incluant en outre un amplificateur de perméation.

4. Formulation de composition chimique selon l'une quelconque des revendications 1 à 3, incluant en outre des ligands sur la surface liposomale.

5. Formulation de composition chimique selon la revendication 4,
dans laquelle lesdits ligands sont le monosialoganglioside ou polyoxyéthylène.

6. Formulation de composition chimique selon l'une quelconque des revendications 1 à 5, dans laquelle ledit liposome a un diamètre initial de moins de ou d'environ 150 nm.

7. Formulation de composition chimique selon l'une quelconque des revendications précédentes destinée à être utilisée dans l'administration d'un médicament à travers une membrane muqueuse en utilisant une formulation de matrice de thermogel ou polymère comprenant les étapes consistant à :
a) fournir une formulation de composition chimique selon l'une quelconque des revendications précédentes ; et
b) appliquer ladite formulation de composition chimique à une membrane muqueuse.

8. Formulation de composition chimique destinée à être utilisée selon la revendication 7, dans laquelle ladite formulation est utilisée pour traiter une dysplasie cellulaire, un cancer ou d'autres affections.
